# EUROPEAN PATENT APPLICATION

(11) **EP 4 796 046 A1**
(43) Date of publication of application: **26.08.2026**
(21) Application number: 25158997.4
(22) Date of filing: 20.02.2025
(51) Int. Cl.: A61B 8/06, A61B 8/08, A61B 8/00, G01S 15/89

(54) **CARDIAC ULTRASOUND IMAGE PROCESSING**

(71) Applicant: Koninklijke Philips N.V., 5656 AE Eindhoven (NL)
(72) Inventor: CLARK, David Wesley, Eindhoven (NL); SHRESTHA, Mona, Eindhoven (NL); RIVERA, Lydia E., 5656AG Eindhoven (NL)
(74) Representative: Philips Intellectual Property & Standards

(57) **Abstract**

A method for processing cardiac ultrasound color flow image data. The method includes receiving color flow data indicative of blood flow in a cardiac region of a subject, generating an initial color flow image (82) for the cardiac region based on the received color flow data, deriving one or more flow parameters from the received color flow data, applying a target area determination operation (18) to determine a target area in the initial image based on the values of the color flow parameters corresponding to each image pixel, creating an image mask (72) comprising mask pixels corresponding to image pixels and having values dependent on whether the corresponding image pixels belong to the target area, obtaining a modified color flow image (84) by selectively processing the initial color flow image using the image mask, and displaying the modified color flow image on a display device (44).

## Description

### FIELD OF THE INVENTION

The present invention relates to processing cardiac ultrasound color flow image data for visualization of target flow regions within the heart.

### BACKGROUND OF THE INVENTION

Cardiac color flow imaging is a commonly used ultrasound technique for visualizing and assessing blood flow within the heart, for example for detecting and evaluating regurgitant jet flow associated with valvular heart disease. This imaging modality combines traditional grayscale ultrasound with color-coded velocity information to provide real-time visualization of blood flow patterns. The ability to accurately visualize and quantify regurgitant jets is important for diagnosing and managing conditions such as mitral regurgitation.

However, current cardiac color flow imaging techniques face several challenges in clearly depicting regurgitant jets. The strong signals from normal forward flow through heart chambers and valves can often obscure the smaller, more turbulent regurgitant jets. Additionally, the dynamic nature of cardiac motion and variations in flow velocities throughout the cardiac cycle make it difficult to consistently capture and display regurgitant jets. These factors can lead to suboptimal visualization and potential misinterpretation of the severity of valvular regurgitation.

It has been appreciated that an enhanced cardiac color flow imaging technique is needed that overcomes one or more of these problems.

### SUMMARY OF THE INVENTION

The invention is defined by the claims.

According to an aspect of the invention, there is provided a method for processing cardiac ultrasound color flow image data. The method comprises: receiving color flow data indicative of blood flow in a cardiac region of a subject; generating an initial color flow image for the cardiac region based on the received color flow data, the initial color flow image comprising a plurality of image pixels; deriving one or more flow parameters from the received color flow data; applying a target area determination operation comprising determining a target area in the initial image based on the values of the flow parameters corresponding to each image pixel; creating an image mask comprising a plurality of mask pixels, each mask pixel corresponding to an image pixel and having a value dependent on whether the corresponding image pixel belongs to the target area; obtaining a modified color flow image by selectively processing the initial color flow image using the image mask; and displaying the modified color flow image on a display device.

This method allows for enhanced visualization of specific target areas within cardiac ultrasound color flow images, such as regurgitant jets, by selectively processing different regions of the image based on derived flow parameters. This can improve diagnostic capabilities and ease of interpretation for clinicians.

By way of example, "selectively processing" may mean applying different image processing operations or parameters to different regions or pixels of an image based on certain criteria or conditions. It may comprise selectively varying one or more parameters of an image processing operation applied to the image for different pixels within the image.

In some embodiments, the target area determination operation comprises applying a transformation to the values of the flow parameters corresponding to each image pixel. In some embodiments, an output of the target area determination operation is an output value for each image pixel dependent on a likelihood of the image pixel being within the target area.

The one or more flow parameters may have values varying as a function of location in the cardiac region. In some embodiments, the one or more flow parameters may comprise at least one of: an estimated flow velocity at a location in the cardiac region, an estimated turbulence at a location in the cardiac region, an amplitude of a flow signal at a location in the cardiac region, a position of a location relative to a cardiac anatomy in the cardiac region, or a position of a location relative to a user-defined region of interest within the cardiac region.

These flow parameters are particularly useful for delineating different flow regions. For example, regions of higher velocity, higher turbulence and/or lower signal amplitude are characteristic of regurgitant jets.

With regards to the user-defined region of interest, in state of the art color flow imaging systems, the system permits a user to define a 'color region-of-interest' within a broader cardiac area which is being imaged using B-mode. Color flow data is then acquired corresponding only to the color region of interest.

In some embodiments, the target area may comprise a high velocity, turbulent flow region. In some embodiments the target area may comprise a low amplitude region.

In some embodiments, the target area determination operation may further comprise retrieving from a memory a stored image mask corresponding to an identified target area of a previously acquired image, wherein the target area in the initial image is determined further based on mask pixel values of the stored image mask corresponding to each image pixel of the initial image. This may allow for temporal consistency in target area identification across multiple image frames or acquisitions, potentially improving the stability and reliability of the visualization.

In some embodiments, the target area may comprise a regurgitant jet area. The regurgitant jet area may be a mitral valve regurgitant jet area.

In some embodiments, selectively processing the initial color flow image may comprise selectively attenuating image pixels based on corresponding mask pixel values. Selectively attenuating image pixels may comprise applying a greater attenuation to image pixels corresponding to mask pixels indicating areas outside of the target area.

This selective attenuation can enhance the visibility of the target area (e.g., regurgitant jet) by reducing the prominence of non-target areas, potentially making it easier for clinicians to identify and assess areas of clinical interest.

By way of example, "selectively attenuating" may mean reducing the relative visibility of the original image pixel in the modified color flow image. By way of example, it may comprise reducing an intensity or prominence of specific image pixels or regions to a variable degree, where the degree of attenuation is determined based on the value of the corresponding mask pixel(s). Reducing an intensity, prominence or visibility may comprise changing a value of a visual characteristic of the pixel in the image, e.g. intensity, color, transparency, etc.

Selectively processing the initial color flow image may comprise attenuating image pixels outside the target area more than pixels inside the target area, so that the visibility of the target area is enhanced.

In some embodiments, the target area determination operation may comprise determining a likelihood of each image pixel of the initial image belonging to the target area, and creating the image mask may comprise assigning mask values to pixels based on their likelihood of belonging to the target area.

In some embodiments, the mask pixel values may span continuously between 0 and 1. In other words, the mask may be a soft mask.

Using a continuous range of mask values allows for a more nuanced and gradual transition between target and non-target areas.

In some embodiments, the selectively processing the initial color flow image comprises selectively attenuating image pixels based on corresponding mask pixel values, and wherein an amount of attenuation applied to each pixel is based on a corresponding mask value.

In some embodiments, the selective processing of the initial color flow image may comprise applying spatial filtering, wherein a degree of spatial filtering applied to each pixel is determined based on a corresponding mask value.

In some embodiments, the spatial filtering comprises smoothing, and wherein spatially filtering the initial color flow image comprises applying a greater degree of smoothing to image pixels corresponding to mask pixels indicating areas outside the target area.

This approach allows for adaptive spatial filtering, potentially enhancing image quality by applying stronger smoothing to areas of less clinical interest while preserving detail in target areas.

In some embodiments, the selective processing may have a configurable modification intensity level, wherein the modification intensity level defines a degree of modification of pixel values of the initial color flow image to be applied as a function of the mask value corresponding to a given pixel value. In other words, the intensity of the selective processing is adjustable.

In some embodiments, the method further comprises receiving a user control input from a user a control element and modulating the modification intensity level in dependence upon the user control input. Providing user control over the modification intensity allows clinicians to adjust the visualization to their preferences or the specific requirements of different clinical scenarios.

In some embodiments, the target area determination operation comprises applying a transformation to the values of the flow parameters corresponding to each image pixel. In some embodiments, the transformation applied by the target area determination operation may comprise using a machine learning model trained on labeled cardiac ultrasound color flow images. In further embodiments, the transformation applied by the target area determination operation may comprise combining two or more of the flow parameters using a mathematical function.

In some embodiments, the method may further comprise automatically adjusting a continuous wave Doppler cursor position based on the determined target area. This feature can streamline the workflow for clinicians by automatically positioning the Doppler cursor in the area of interest, potentially saving time and improving the accuracy of Doppler measurements.

In some embodiments, the method may further comprise quantifying at least one characteristic of the target area based on the image mask. In some embodiments, the characteristic comprises at least one of: a regurgitant flow volume, an orifice area, or a severity grade of valvular regurgitation.

A further aspect of the invention provides a computer program product comprising computer program code configured, when run on a processor, to cause the processor to perform a method in accordance with any embodiment detailed in this disclosure or in accordance with any claim.

A further aspect of the invention is a processing device comprising one or more processors configured to perform a method in accordance with any embodiment detailed in this disclosure or in accordance with any claim.

A further aspect of the invention is a system. The system comprises: a processing device in accordance with any embodiment outlined herein or in accordance with any claim; and an ultrasound imaging system operable to generate ultrasound color flow data and operatively coupled with the processing device; and a display device operatively coupled with the processing device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 depicts a flowchart of an example method for processing cardiac ultrasound color flow image data in accordance with one or more embodiments of the invention;
Fig. 2 depicts a block diagram of an example system for processing cardiac ultrasound color flow image data in accordance with one or more embodiments of the invention;
Fig. 3 shows a series of grayscale images representing flow parameter maps representative of different flow parameters as a function of position;
Fig. 4 illustrates a block diagram of an example procedure for generating an image mask in accordance with one or more embodiments of the invention;
Fig. 5 illustrates an example operation for applying selective processing to pixels of an initial image based on a created mask;
Fig. 6 illustrates user-configurable intensity of image modification in accordance with one or more embodiments of the invention;
Fig. 7 illustrates a series of ultrasound images demonstrating different levels of non-jet attenuation achievable using an example embodiment of the invention; and
Fig. 8 illustrates a series of ultrasound images demonstrating different types of smoothing applied to a non-jet region using an example embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Cardiac ultrasound color flow imaging is a valuable diagnostic tool for assessing blood flow in the heart. However, traditional color flow imaging techniques can sometimes obscure important features, such as regurgitant jets, due to the presence of strong signals from normal forward flow.

Embodiments of the present invention are based on processing cardiac ultrasound color flow image data to enhance visualization of clinically relevant flow patterns. Embodiments of the invention provide a method comprising receiving color flow data indicative of blood flow in a cardiac region and generating an initial color flow image based on this data. One or more flow parameters are derived from the received color flow data. These parameters may include for example velocity, turbulence, signal amplitude, and/or spatial characteristics of the flow. A target area determination operation is applied to identify one or more regions of interest within the image, such as areas likely to contain regurgitant jets. This operation uses the derived flow parameters to create an image mask. The image mask comprises a plurality of mask pixels, each corresponding to an image pixel and having a value dependent on whether the corresponding image pixel belongs to the target area. The initial color flow image is selectively processed using the image mask to obtain a modified color flow image. This selective processing may involve attenuating non-target areas or applying different levels of spatial filtering to different regions of the image. The resulting modified color flow image may be displayed on a display device, providing an enhanced view of the target areas.

In at least one set of advantageous embodiments, the method may also include permitting a user to adjust one or more user-configurable settings to adjust the intensity of the selective processing, allowing clinicians to optimize the image for particular diagnostic requirements.

By selectively processing the color flow image data, preferential visualization of clinically relevant flow patterns can be achieved while reducing visual clutter from less diagnostically important regions. This enhanced visualization can aid in the detection and assessment of cardiac conditions such as valvular regurgitation.

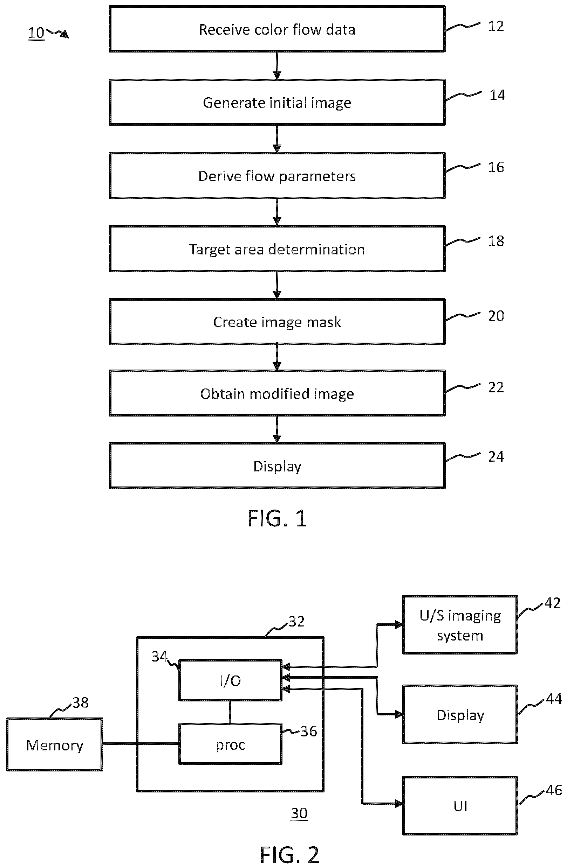

Fig. 1 illustrates a method 10 for processing cardiac ultrasound color flow image data. The method 10 comprises a sequence of steps that may be implemented as computer program code configured to be run on a processor.

The method 10 comprises receiving 12 ultrasound color flow data indicative of blood flow in a cardiac region of a subject. The color flow data typically contains information about blood velocity within the heart and its major vessels. This data may be acquired using Doppler ultrasound techniques. The data may be two-dimensional or three-dimensional data, depending on the ultrasound acquisition mode used. The cardiac region may encompass various structures of the heart, including chambers, valves, and/or major blood vessels. Of particular interest are areas where abnormal flow patterns might occur, such as regions near heart valves where regurgitant jets may be present in cases of valvular disease.

The method 10 further comprises generating 14 an initial color flow image for the cardiac region based on the received color flow data. The initial color flow image comprises a plurality of image pixels representing the blood flow in the cardiac region.

The method 10 further comprises deriving 16 one or more flow parameters from the received color flow data. The one or more flow parameters may have values varying as a function of location in the cardiac region. By way of example, the parameters may include an estimated flow velocity, an estimated turbulence, an amplitude of a flow signal, a position relative to a cardiac anatomy, or a position relative to a user-defined region of interest. These parameters may be computed using one or more predetermined functions or algorithms applied to the received ultrasound data. By way of example, an estimated turbulence at a given location may be quantified using a variance of the Doppler spectrum at a given location. An amplitude of flow signal might be calculated as the power of the Doppler signal. With regards to determining a position relative to cardiac anatomy, this parameter may be determined by applying image segmentation techniques to identify cardiac structures, followed by coordinate mapping to relate flow data positions to anatomical landmarks. Machine learning algorithms may be employed for automated segmentation.

With regards to determining position relative to a user-defined ROI, this may be computed using simple coordinate geometry comprising calculating the distance between each pixel and the boundaries of the user-defined region of interest. In some implementations, these parameters may be combined or weighted to create composite metrics for target area determination.

The method 10 furthermore comprises applying 18 a target area determination operation. This operation determines a target area in the initial image based on the values of the flow parameters corresponding to each image pixel. The target area may represent one or more regions of particular interest within the cardiac image. The target area may comprise a regurgitant jet area.

The method further comprises creating 20 an image mask. The image mask comprises a plurality of mask pixels, with each mask pixel corresponding to an image pixel in the initial color flow image. The value of each mask pixel is dependent upon whether the corresponding image pixel belongs to the determined target area.

It is noted that the target area determination operation and the mask creation may in some embodiments be performed together. For example, the process for determining the target area may automatically result in creation of an image mask, or the creation of the image mask may inherently achieve determination of the target area. In some embodiments, the target area determination operation comprises applying a transformation to the values of the flow parameters corresponding to each image pixel, and wherein values for the image mask pixels are computed based on the output of the transformation.

The method 10 further comprises obtaining 22 a modified color flow image based on electively processing the initial color flow image using the created image mask. The selective processing may enhance or modify certain areas of the image based on the mask values. The selective processing may change a relative visibility of each pixel in the image in dependence on the mask value corresponding to the pixel. The selective processing may change a relative visibility of each pixel in the image in dependence upon whether the pixel falls within or outside the target area.

The method may further comprise displaying 24 the modified color flow image on a display device.

The sequence of steps in the method 10 may be implemented as a computer program product comprising computer program code. When run on a processor, this computer program code causes the processor to perform the described method.

Fig. 2 illustrates a block diagram of an example system for processing cardiac ultrasound color flow image data. The system includes a processing device 32, an ultrasound imaging system 42, and a display device 44. The system may further comprise a user interface 46.

The processing device 32 comprises an input/output interface 34 and one or more processors 36. The processing device 32 is configured to perform the method 10 described in relation to Fig. 1.

The ultrasound imaging system 42 is operatively coupled with the processing device 32. The ultrasound imaging system 42 is operable to generate ultrasound color flow data, which may be received by the processing device 32 through the input/output interface 34.

The display device 44 is operatively coupled with the processing device 32. The display device 44 may be used to display the modified color flow image produced by the selective processing of the initial image using the mask.

The system optionally includes a user interface 46 operatively coupled with the processing device 32. The user interface 46 may allow a user to interact with the system and control one or more elements of the image processing. As will be explained in more detail later, the user interface 46 may permit a user to adjust an intensity level of the image modification performed using the mask.

The system optionally includes a memory 38 operatively coupled with the processing device 32. As will be explained in more detail later, the processing device 32 may retrieve from the memory 38 a stored image mask corresponding to an identified target area of a previously acquired image.

The one or more processors 36 of the processing device 32 are configured to execute the steps of the method 10.

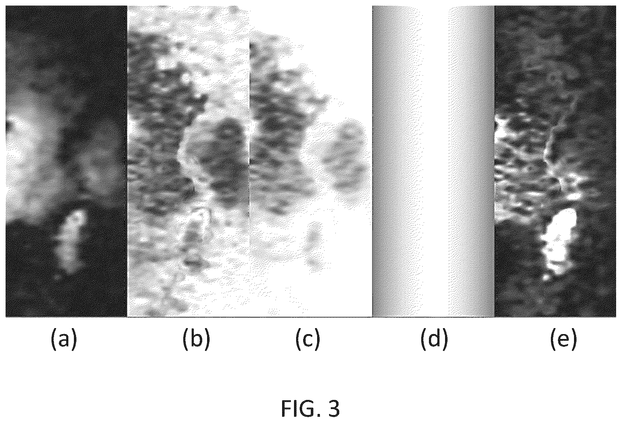

Fig. 3 illustrates a series of grayscale images representing spatial maps of each of a number of different example flow parameters which may be used in generating an image mask in accordance with one or more embodiments. The figure shows five images labeled (a) to (e).

Image (a) displays an absolute velocity map, showing the magnitude of flow velocities in an example cardiac region as a function of position in the cardiac region. The brightness of each pixel in this image corresponds to the absolute value of the estimated flow velocity at that location. Areas of high velocity, which may indicate potential jet regions, appear brighter in this map.

Image (b) presents a turbulence map, indicating areas of turbulent flow within the cardiac region. The brightness in this image corresponds to the estimated turbulence at each location. Regions of high turbulence, often associated with regurgitant jets, appear as brighter areas in this map.

Image (c) shows an amplitude weight factor map. This map represents a function of the amplitude or power of the flow signal at each location. The brightness in this image corresponds to the strength of the flow signal, with weaker signals appearing brighter.

Image (d) depicts a lateral position factor map, representing a lateral position of each location in the cardiac region relative to a user-defined region of interest within the cardiac region. The brightness in this image corresponds to degree of lateral central alignment of a given location within the user-defined region of interest, with the brightest pixel representing exact centering of the relevant location (represented by the relevant pixel of the image) within the user-defined region of interest. This map may be used to incorporate spatial information into the jet mask generation process, potentially giving more weight to centrally located flow patterns.

Image (e) presents an example image mask obtained by combining the flow parameters represented in images (a)-(d). This mask represents the likelihood of each pixel belonging to a predefined target area, such as a regurgitant jet area. In this example, the brightness of each pixel in this mask corresponds to the likelihood of that pixel being part of the target area.

In some examples, the target area determination operation may use such flow parameters, having values which vary as a function of position, as inputs to determine regions of interest within the initial cardiac ultrasound color flow image. In some examples, the operation may apply a transformation to the values of the flow parameters corresponding to each image pixel. In some implementations, this transformation may involve combining two or more of the flow parameters using a mathematical function. In some implementations, the transformation may comprise use of deep learning pattern recognition trained with manually-labeled images to produce the soft mask.

The one or more flow parameters used in the target area determination operation have values varying as a function of location in the cardiac region. These parameters may include an estimated flow velocity, an estimated turbulence, an amplitude of a flow signal, a position relative to a cardiac anatomy, or a position relative to a user-defined region of interest. By combining these parameters, the system may effectively identify and highlight areas of clinical interest, such as regurgitant jet areas, within the cardiac ultrasound color flow image.

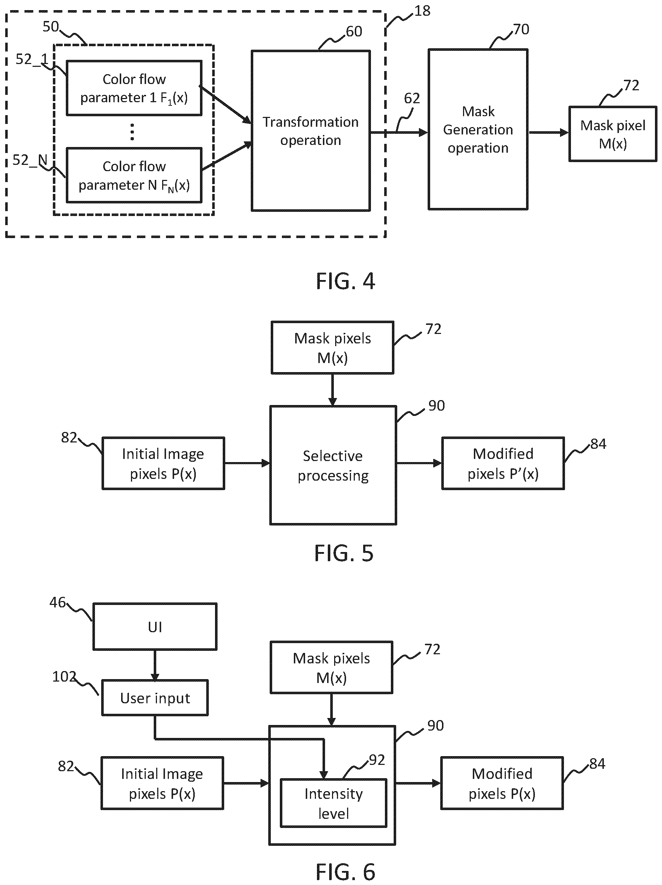

Fig. 4 illustrates a block diagram of an example procedure for generating an image mask in accordance with one or more embodiments of the invention.

As noted previously a set 50 of one or more flow parameters are derived from the received color flow data. The set of one or more flow parameters 50 comprises *N* flow parameters: a first flow parameter 52_1 through to an Nth flow parameter 52_N. These parameters may be derived from the color flow data received in the step of receiving 12 of the method 10. Mathematically, an nth flow parameter is represented as Fₙ(x), where x represents a pixel position co-ordinate in the color flow image and Fₙ represents the nth flow parameter, which is a function of *x.*

In the illustrated example, as part of the target area determination operation 18, the set of one or more flow parameters 50 serves as input to a transformation operation 60. The transformation operation 60 applies a transformation to the values of the flow parameters corresponding to each image pixel. In some examples, the transformation operation 60 may use a trained machine learning model. In some examples, the transformation operation may comprise combining two or more of the flow parameters using a mathematical function.

The transformation operation 60 produces an output 62. The output of the transformation operation may be a parameter value corresponding to each pixel location, x, which for example represents a likelihood the respective image pixel of the initial image belonging to the target area. The output 62 may serve as input to a mask generation operation 70. The mask generation operation 70 may create an image mask by assigning mask values to pixels based on their likelihood of belonging to the target area. In some embodiments, the mask values may span continuously between 0 and 1, allowing for a soft mask that represents varying degrees of likelihood.

The mask generation operation 70 generates a set 72 of mask pixel values as its final output, at least one mask pixel value for each pixel location, x. These mask pixels 72 correspond to the image pixels of the initial color flow image. The mask may be represented mathematically as M(x), where each value of M is the mask pixel value at pixel location, x.

Optionally, in some examples, the target area determination operation 18 may utilize one or more additional inputs. For example, in some embodiments, the target area determination operation 18 may retrieve from a memory, such as for example memory 38 in the system of Fig. 1, a stored image mask corresponding to an identified target area of a previously acquired image. The target area in the current initial image may be determined based on both the current flow parameters and the mask pixel values of the stored image mask corresponding to each image pixel of the initial image. This approach may enhance temporal consistency in target area identification across multiple image frames or acquisitions, potentially improving the stability and reliability of the visualization over time.

The mask pixels 72 produced by the mask generation operation 70 form the image mask, M(x). The image mask is subsequently used to selectively process the initial color flow image and obtain the modified color flow image.

Fig. 5 schematically illustrates a block diagram of a selective image processing operation performed after creating the image mask, M(x).

The method comprises receiving initial image pixel data 82 for the initial image, this comprising initial image pixel values P(x), each pixel value, P, corresponding to a pixel spatial location, x, in the initial image. In this disclosure, pixel spatial location x may represent a 2D or 3D spatial co-ordinate. In other words, spatial location x may be defined by two or three co-ordinate values, e.g. x = [x, y] or x=[x, y, z]. In other words, x may be understood as a position vector or matrix. Alternatively, x may be represented by a single-value pixel index.

A selective processing operation 90 is applied, comprising processing the initial image pixels P(x) using the mask pixels M(x) to generate modified image data 84 comprising modified image pixels P'(x) as output. The modified image pixels 84 form a modified color flow image.

In some embodiments, the selective processing 90 may comprise selectively attenuating the initial image pixels 82 based on corresponding mask pixel values, M(x). An amount of attenuation applied to each initial image pixel, P(x), may be based on a corresponding mask value, M(x), for the corresponding pixel location. In some examples, the selective processing 90 comprises applying a greater attenuation to initial image pixels P(x) corresponding to mask pixels M(x) indicating areas outside of the target area.

The selective processing 90 may additionally or alternatively comprise applying spatial filtering to the initial image pixels P(x). A degree of spatial filtering applied to each initial image pixel P(x) may be based on a corresponding mask pixel value, M(x). The spatial filtering may comprise smoothing. In some examples, the selective processing 90 applies a greater degree of smoothing to initial image pixels P(x) corresponding to mask pixels M(x) indicating areas outside the target area. For example, the method may comprise applying stronger smoothing to areas with lower (/higher) mask values, which may correspond to regions outside the target area. Conversely, areas with higher (/lower) mask values, likely corresponding to the target area, may receive less aggressive smoothing to preserve important flow details. This adaptive spatial filtering approach may help enhance the overall image quality while maintaining the visibility of clinically relevant features.

One simple approach to applying a variable smoothing is to use the soft jet mask to variably blend the output of a strong spatial smoothing filter applied to the initial image with a less smoothed version of the initial image. Another way is to use the soft jet mask to select different spatial filter coefficients (span or bandwidth) for each point in the image.

By selectively processing the initial image pixels 82 based on the mask pixels 72, the image processing system enhances the visibility of target areas, such as regurgitant jets, in the cardiac ultrasound color flow image. The modified image pixels P'(x) produced by the selective processing 90 form the modified color flow image for display on the display device 44.

The selective processing 90 of the initial image 82 to obtain the modified image 84 can be represented mathematically as follows.

The mask pixel values M(x) may be defined as a function of the flow parameters, Fₙ(x), derived from the initial color flow image. Mathematically, this can be expressed as: $M \left(x\right) = f \left({F}_{1}\left(x\right),{F}_{2}\left(x\right),…,{F}_{N}\left(x\right)\right)$ where *F*₁(*x*)*,F*₂(*x*)*,..., F_{N}*(*x*) represent the values of the set of one or more flow parameters at location x, and f is a function that maps these parameters to a mask pixel value.

The transformation operation 60 applied during the apply target area determination operation 18 can be represented as this function f. In some examples, the function f may be a simple linear combination of the input parameters: $f \left({F}_{1}\left(x\right),{F}_{2}\left(x\right),…,{F}_{N}\left(x\right)\right) = {w}_{1} {F}_{1} \left(x\right) + {w}_{2} {F}_{2} \left(x\right) + … + {w}_{N} {F}_{N} \left(x\right)$ where *w*₁, *w*₂, ..., *w_{N}* are weighting factors for each parameter, Fₙ. In some embodiments, the weighting factors may be configurable, optionally in dependence upon a user input or user-defined setting.

In other examples, the function f may be a more complex non-linear function, such as a sigmoid function: $f \left({F}_{1}\left(x\right),{F}_{2}\left(x\right),…,{F}_{N}\left(x\right)\right) = \frac{1}{1 + {e}^{- \left({w}_{1}{F}_{1}\left(x\right)+{w}_{2}{F}_{2}\left(x\right)+…+{w}_{N}{F}_{N}\left(x\right)\right)}}$

In some embodiments, the weighting factors, wₙ, may be configurable, optionally in dependence upon a user input or user-defined setting.

The mask generation operation 70 uses the output of the transformation operation, 60, comprising transformation function, *f*, to create the mask M. In some examples, each mask pixel value, M(x) is simply set equal to the output 62 of the transformation function applied to the flow parameter values for pixel, x. This is the case in the example equation set out previously. In other examples, a further conversion operation may be applied to convert the transformation function output for pixel, x, into a mask value. For example, the mask pixel values M(x) may be continuous values between 0 and 1 (or some other defined value range), and thus a conversion operation may normalize or otherwise convert the transformation operation 60 output 62 into mask values M(x). In some examples, the transformation function may be such that the mask value for each pixel, x, represent the likelihood of the pixel at location x belonging to the target area.

The method comprises using the mask, M, to selectively process the initial image pixels, P(x). This selective processing can be mathematically represented as: $P ′ \left(x\right) = g \left(P\left(x\right),M\left(x\right)\right)$ where P(x) represents the initial image pixels, P'(x) represents the modified image pixels, and g is a function that defines the selective processing operation.

For example, if the selective processing involves selective attenuation of image pixels, the function g might be: $P ′ \left(x\right) = P \left(x\right) ⋅ \left(1-α⋅\left(1-M\left(x\right)\right)\right)$ where *α* is an attenuation factor. By way of example, here the mask value, M(x), for each pixel location, x, may take a value between 0 and 1, where 0 represents zero likelihood of the pixel at location x being within the target area, and 1 represents a 100% likelihood of the pixel at location x being within the target area. Accordingly, when the mask value M(x) is at 1, no attenuation is applied, and the modified pixel value P'(x) remains identical to the original pixel value P(x). When the mask value M(x) is at 0, maximum attenuation is applied, with a strength that depends upon *α*, wherein the modified pixel value P'(x) is set at
*P'*(*x*) *= P*(*x*) · (1 - *α*). If the mask value M(x) has a value between 0 and 1, then the modified pixel value P'(x) will be set at a value less than the original pixel value P(x) by an amount dependent on M(x) and upon the attenuation factor *α*.

The attenuation factor, *α*, may take a value between 0 and 1.

The attenuation factor, *α*, may be set at a fixed value or may be configurable. For example, and as will be explained in more detail later, the selective processing 90 may have a configurable modification intensity level, wherein the modification intensity level defines a degree of modification of pixel values P(x) of the initial color flow image to be applied as a function of the mask value M(x) corresponding to a given pixel value, P(x). In this case, the attenuation factor, *α*, may be defined as a function of the modification intensity level. The modification intensity level may be configurable in dependence upon a user input.

If the selective processing 90 involves spatial filtering, the function g might involve a convolution operation: $P ′ \left(x\right) = \left(P\left(x\right)∗{K}_{M \left(x\right)}\right)$ where *K*_{*M*(*x*)} is a spatially varying convolution kernel that depends on the mask value M(x).

The dependency of the convolution kernel on M(x) may be fixed or may be configurable. It may vary in dependence upon a configurable modification intensity level. The modification intensity level may be configurable in dependence upon a user input.

By way of illustration, one implementation of the selective processing operation described above might be briefly summarized as follows. For each pixel, P(x), at pixel location, x, of the initial image, a transformation function is applied to the values of the set of flow parameters, [*F*₁(*x*)*, F*₂(*x*)*,..., F_{N}*(*x*)], corresponding to the pixel location x to transform the flow parameters into a single-value target area (likelihood) parameter, indicative of a likelihood of the pixel location x belonging to the target area. This transformation represents the target area determination operation 18 discussed previously. A conversion function or operation might then be applied to convert the target area (likelihood) parameter into a mask value corresponding to the pixel, optionally wherein the conversion function has output values spanning continuously between 0 and 1, resulting in a soft mask. This corresponds to the step of creating 20 an image mask referred to previously. Selective processing may be applied to the initial image pixels P(x). This may for example be in the form of an attenuation function, wherein the attenuation at each pixel location x is dependent upon the soft mask value M(x) corresponding to the pixel location x. Additionally or alternatively, this may be in the form of a smoothing filter function, wherein the degree of smoothing at each pixel location x is dependent upon the soft mask value M(x) corresponding to the pixel location, x. This selective processing corresponds to the step of obtaining 22 the modified color flow image referred to previously.

By selectively processing the initial color flow image using a target area determination operation and an image mask, the method enhances the visibility of clinically relevant features such as regurgitant jets while reducing visual clutter from less diagnostically important regions.

Although in the example described above, the transformation function used in the target area determination operation comprises a weighted sum of the flow parameters, in other embodiments, the transformation applied to the values of the flow parameters corresponding to each image pixel may involve using a machine learning model trained on labeled cardiac ultrasound color flow images to identify regions of clinical significance such as areas exhibiting characteristic flow patterns such as high-velocity jets, or turbulence.

The machine learning model used for this purpose may be a convolutional neural network (CNN) or a hybrid architecture combining CNN layers with recurrent neural network (RNN) components. The model may be configured to accept as input a multi-channel representation of the ultrasound image, where each channel corresponds to one of the *N* flow parameters, Fₙ(x). The CNN may comprise one or more feature extraction layers which extract spatial features from the input image by applying convolutional filters configured to identify patterns associated, for example, with flow anomalies, gradients, and vessel structures. One or more fully connected layers may map the extracted features to high-level representations, with the final representation corresponding to a target area prediction.

The final output may comprise a pixel-wise map, with one value per pixel representing a confidence score indicative of a likelihood of the pixel being part of the target area. The model may be trained in a supervised learning process, using a dataset of labeled cardiac ultrasound color flow images. The dataset used in training might comprise a plurality of cardiac ultrasound images captured in various clinical scenarios, and wherein each image is associated with multiple flow parameters defined as separate channels. Expert clinicians may annotate the images to identify the relevant target area in each image. The model may then be trained to classify image pixels according to whether or not the pixel falls within the target area, with an associated confidence score for each pixel.

With reference to Fig. 6, in some embodiments, the selective processing 90 may have a configurable modification intensity level 92, wherein the modification intensity level defines a degree of modification of pixel values, P(x), of the initial color flow image 82 to be applied as a function of the mask 72 value, M(x), corresponding to a given pixel value.

Fig. 6 shows a block diagram illustrating an example implementation of this functionality.

A modification intensity level setting 92 is associated within the selective processing operation 90. The modification intensity level 92 defines a degree of modification to be applied to the pixel values of the initial image pixels 82 as a function of the corresponding mask pixel values. The selective processing 90 has a configurable modification intensity level 92, allowing for adjustable processing of the image.

In this example, the system includes a user interface 46 permitting a user to provide a user control input 102.

The method 10 comprises receiving the user control input 102 from a user control element of the user interface 46. In some embodiments, the user control element may comprise a slider or toggle control element permitting a user to dynamically adjust the level of processing applied to the image. By adjusting the modification intensity, users may fine-tune the balance between enhancing the visibility of the target area and preserving contextual information from surrounding regions. In some embodiments, the user control input 102 may be incorporated as part of a color gain (display threshold) control of the ultrasound imaging system 42. This integration allows for intuitive user control without introducing additional interface elements.

The method 10 comprises modulating the modification intensity level 92 in dependence upon the user control input 102. As the user adjusts the control, the modification intensity level 92 changes accordingly, influencing the degree of selective processing applied to the initial image pixels 82.

The initial image 82 pixel P(x) and mask 72 pixels, M(x), are input to the selective processing operation 90 which selectively processes initial image pixels P(x) using the mask pixels M(x) to generate the modified image 84 having modified image pixels P'(x).

By providing user control over the modification intensity level 92, the system allows for customization of the image processing. Users may adjust the intensity of the selective processing to optimize the visibility of target areas, such as regurgitant jets, while maintaining appropriate levels of detail in other regions of the cardiac color flow image.

With regards to the user control element of the user interface 46, in some embodiments, the user control element may be implemented by a color gain control e.g. a color gain knob. In other words, the control of the modification intensity level may be integrated with a color gain control element comprised by the ultrasound imaging system.

This approach to implementing the user control of the modification intensity level 92 may allow for intuitive user control without introducing additional interface elements.

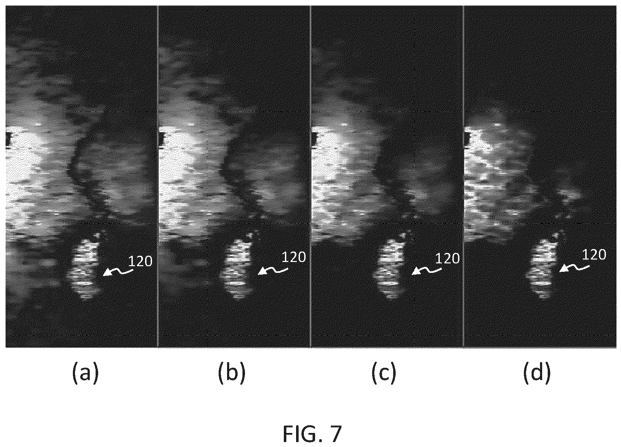

The variable modification intensity level is illustrated in Fig. 7 which shows a series of example modified color flow images, resulting from application of an example embodiment of the method 10 to an initial image of a mitral jet with left ventricle outflow. In this example, the target region is the mitral jet flow region, wherein the initial image has been selectively processed to achieve attenuation of image regions which are outside of the detected jet flow region. The jet flow region is indicated by arrow 120. Each image represents a result of applying the mask to the initial image with a different intensity level of attenuation of the non-jet region. Image (a) shows the original image with zero attenuation. Image (b) shows a modified image with non-jet attenuation of 10dB. Image (c) shows a modified image with non-jet attenuation of 20dB. Image (d) shows a modified image with non-jet attenuation of 30dB. As can be seen, the forward chamber flow is attenuated while the regurgitant jet flow is almost unaffected.

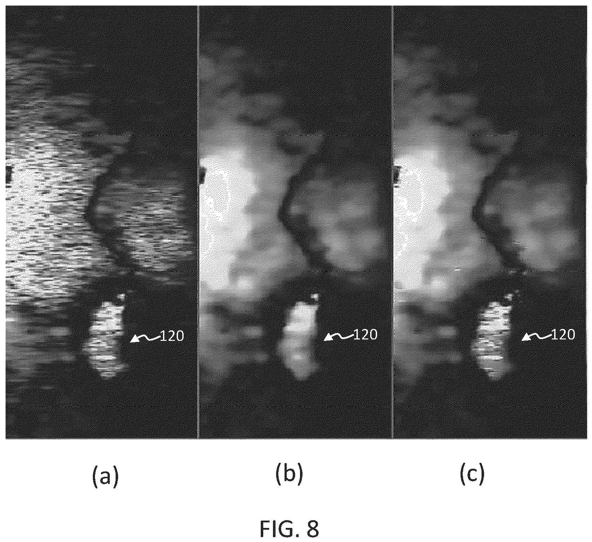

Fig. 8 illustrates application of example embodiments of the method 10 wherein the initial image has been selectively processed to achieve smoothing of image regions which are outside of the detected jet flow region 120. Image (a) shows the original image with zero smoothing. Image (b) shows a modified image with smoothing applied evenly to the whole image. Image (c) shows a modified image selectively processed with an embodiment of the invention, wherein the jet region 120 is left unsmoothed with high resolution, and the non-jet regions have been smoothed.

As previously discussed, one advantageous application for embodiments of the present invention is for enhancement of visibility of a regurgitant jet flow region and selective suppression of a non-jet flow region.

In this context, a jet flow region is typically associated with high-velocity, high-turbulence flow regions, while a non-jet-flow region is typically associated with lower velocity, laminar flow regions.

Thus, in accordance with one or more embodiments, the determining of a target area in the initial image based on the values of the flow parameters corresponding to each image pixel may comprise determining that pixels located at a location associated with higher velocity have a higher probability of being within the target area than pixels located at a location associated with a lower velocity and/or determining that pixels located at a location associated with higher turbulence have a higher probability of being within the target area than pixels located at a location associated with a lower turbulence. For example, the transformation function 60 may be configured to make this distinction.

In some embodiments, the method may further comprise performing quantification of one or more characteristics of the target area based on the image mask. These characteristics may include one or more of: regurgitant flow volume, orifice area, or severity grade of valvular regurgitation. By providing quantitative metrics derived from the enhanced image data, the method supports more objective and standardized assessment of cardiac conditions.

In some embodiments, the method may comprise automatically adjusting a continuous wave (CW) Doppler cursor position based on the determined target area. The cursor position refers to the spatial location and orientation of the continuous wave (CW) Doppler sampling line within the imaging region of interest, as displayed on a graphical user interface of the system. In the context of a color flow imaging system, the cursor position serves as a visual and functional indicator of the local area from which Doppler signal data is being collected and analyzed. This automation streamlines the workflow for clinicians, potentially improving the accuracy and efficiency of Doppler measurements in areas of interest such as regurgitant jets.

In addition to adjusting the Doppler cursor position, in some embodiments, the method may comprise automatically configuring one or more further imaging parameters based on the determined target area. For example, the method may comprise adjusting the color gain, velocity scale, and/or wall filter settings to enhance the visualization of the identified flow patterns of interest. These automated adjustments may be performed based on the characteristics of the target area, such as its size, velocity range, and/or turbulence levels. By automatically optimizing these parameters, the method may reduce the need for manual adjustments and potentially improve the consistency and efficiency of cardiac ultrasound examinations.

In some implementations, the method may apply different color schemes or transparency levels to highlight the target area while de-emphasizing non-target regions. For instance, the system may use a more saturated or brighter color palette for the target area while applying a desaturated or semitransparent color scheme to the surrounding flow regions. This approach may create a visual highlighting which draws attention to the clinically relevant flow patterns while still providing context from the surrounding flow information.

The modified image may be displayed on a display device 44.

In some embodiments, the method may comprise displaying both the original image and the modified image together on the display screen, for example in side-by-side arrangement or overlay arrangement. This feature may allow clinicians to easily assess the impact of the processing technique and verify that important flow information is preserved while non-target areas are attenuated.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (10) for processing cardiac ultrasound color flow image data, the method comprising:
receiving (12) color flow data indicative of blood flow in a cardiac region of a subject;
generating (14) an initial color flow image (82) for the cardiac region based on the received color flow data, the initial color flow image comprising a plurality of image pixels;
deriving (16) one or more flow parameters (F₁(x)) from the received color flow data;
applying a target area determination operation (18) comprising determining a target area in the initial image based on the values of the flow parameters corresponding to each image pixel;
creating (20) an image mask (M(x)) comprising a plurality of mask pixels, each mask pixel corresponding to an image pixel and having a value dependent on whether the corresponding image pixel belongs to the target area;
obtaining (22) a modified color flow image (84) by selectively processing the initial color flow image using the image mask; and
displaying (24) the modified color flow image on a display device (44).

2. The method of claim 1, wherein each of the one or more flow parameters have values varying as a function of location in the cardiac region, and wherein the one or more flow parameters comprise at least one of: an estimated flow velocity at a location in the cardiac region, an estimated turbulence at a location in the cardiac region, an amplitude of a flow signal at a location in the cardiac region, a position of a location relative to a cardiac anatomy in the cardiac region, or a position of a location relative to a user-defined region of interest within the cardiac region.

3. The method of claim 1 or 2,
wherein the target area determination operation further comprises retrieving from a memory a stored image mask corresponding to an identified target area of a previously acquired image; and
wherein the target area in the initial image is determined further based on mask pixel values of the stored image mask corresponding to each image pixel of the initial image.

4. The method of any preceding claim, wherein the target area comprises a regurgitant jet area.

5. The method of any preceding claim,
wherein selectively processing the initial color flow image comprises selectively attenuating image pixels based on corresponding mask pixel values; and
optionally wherein selectively attenuating image pixels comprises applying a greater attenuation to image pixels corresponding to mask pixels indicating areas outside of the target area.

6. The method of any preceding claim,
wherein the target area determination operation comprises determining a likelihood of each image pixel of the initial image belonging to the target area, and wherein creating the image mask comprises assigning mask values to pixels based on their likelihood of belonging to the target area; and
wherein the mask values span continuously between 0 and 1.

7. The method of claim 6, wherein selectively processing the initial color flow image comprises selectively attenuating image pixels based on corresponding mask pixel values, wherein an amount of attenuation applied to each pixel is based on a corresponding mask value.

8. The method of preceding claim,
wherein selectively processing the initial color flow image comprises applying spatial filtering; and
wherein a degree of spatial filtering applied to each pixel is based on a corresponding mask value, and
optionally wherein the spatial filtering comprises smoothing, and wherein spatially filtering the initial color flow image comprises applying a greater degree of smoothing to image pixels corresponding to mask pixels indicating areas outside the target area.

9. The method of any preceding claim,
wherein the selective processing has a configurable modification intensity level, wherein the modification intensity level defines a degree of modification of pixel values of the initial color flow image to be applied as a function of the mask value corresponding to a given pixel value, and
optionally wherein the method further comprises receiving a user control input from a user a control element and modulating the modification intensity level in dependence upon the user control input.

10. The method of any preceding claim, wherein the target area determination operation comprises applying a transformation to the values of the flow parameters corresponding to each image pixel, and wherein the transformation applied by the target area determination operation comprises using a machine learning model trained on labeled cardiac ultrasound color flow images.

11. The method of any preceding claim, wherein the method further comprises automatically adjusting a continuous wave Doppler cursor position based on the determined target area.

12. The method of any preceding claim, wherein the method further comprises quantifying at least one characteristic of the target area based on the image mask, and optionally wherein the characteristic comprises at least one of: a regurgitant flow volume, an orifice area, and a severity grade of valvular regurgitation.

13. A computer program product comprising computer program code configured, when run on a processor, to cause the processor to perform a method in accordance with any preceding claim.

14. A processing device (32) comprising one or more processors (36) configured to perform a method in accordance with any of claims 1-12.

15. A system (30), comprising:
a processing device (32) in accordance with claim 14;
an ultrasound imaging system (42) operable to generate ultrasound color flow data and operatively coupled with the processing device; and
a display device (44) operatively coupled with the processing device.
